(19) Europäisches Patentamt
European Patent Office
Office européen des brevets

(11) **EP 2 543 719 B1**

(12) **EUROPÄISCHE PATENTSCHRIFT**

(45) Veröffentlichungstag und Bekanntmachung des Hinweises auf die Patenterteilung:
**03.06.2020   Patentblatt 2020/23**

(51) Int Cl.:
**C12M 3/00** (2006.01)   **C12N 5/07** (2010.01)

(21) Anmeldenummer: **12005008.3**

(22) Anmeldetag: **06.07.2012**

(54) **MÄANDER-BIOREAKTOR UND VERFAHREN ZUR DYNAMISCHEN EXPANSION, DIFFERENZIERUNG UND ERNTE VON HÄMATOPOETISCHEN ZELLEN**

MEANDER BIOREACTOR AND METHOD FOR DYNAMIC EXPANSION, DIFFERENTIATION AND HARVEST OF HEMATOPOIETIC CELLS

BIORÉACTEUR AVEC MEANDRE ET PROCÉDÉ D'EXPANSION, DE DIFFÉRENCIATION ET DE RÉCOLTE DYNAMIQUES DE CELLULES HÉMATOPOÏÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.07.2011   DE 102011106914**

(43) Veröffentlichungstag der Anmeldung:
**09.01.2013   Patentblatt 2013/02**

(73) Patentinhaber: **Zellwerk GmbH**
**16727 Eichstädt (DE)**

(72) Erfinder: **Hoffmeister, Hans, Prof. Dr.**
**32549 Bad Oeynhausen (DE)**

(56) Entgegenhaltungen:
**FR-A1- 2 608 169**

- **KLAPPER ET AL.: "Single-pass, closed-system rapid expansion of lymphocyte cultures for adoptive cell therapy", JOURNAL OF IMMUNOLOGICAL METHODS, Nr. 345, 21. April 2009 (2009-04-21), Seiten 90-99, XP002688259,**
- **SCHÜRCH ET AL.: "Experimental evaluation of laminar shear stress on the behavious of hybridoma mass cell cultures, producing monoclonal antibodies against mitochondrial creatine kinase", JOURNAL OF BIOTECHNILOGY, Bd. 7, 1988, Seiten 179-184, XP002688260,**

**Beschreibung**

Mäander- Bioreaktor und Verfahren zur dynamischen Expansion, Differenzierung und Ernte von hämatopoetischen Zellen

Die Erfindung betrifft einen Mäander- Bioreaktor sowie Verfahren für die dynamische und stressfreie Expansion, Differenzierung, Aktivierung, Stimulierung der Gesamtfraktion oder auch einzelner Zellarten der hämatopoetischen Zellen, hämatopoetischen Stammzellen, hämatopoetischen Progenitorzellen darin, wobei der Bioreaktor im Perfusionsbetrieb arbeitet.

Hämatopoetischen Zellen, vorzugsweise Stammzellen werden zur Rekonstruktion des hämatopoetischen Systems und zur Behandlung verschiedener blutbedingter Krankheiten und Störungen eingesetzt (Lu et. al. Critical Reve. Onco / Hematol 22:61-78 1996). In der Vergangenheit wurden große Anstrengungen unternommen, mittels Ex- vivo- Kultivierungssystemen hämatopoetische Zellen, vorzugsweise Stammzellen in ausreichender Menge an Zellen für Transplantationszwecke zu erzeugen.

In der WO 2009139413 wird ein Verfahren für die Herstellung einer Zellmasse beschrieben, die eine cytokin-induzierte Killer Zelle enthält (CIK), wobei die Kultivierung der Zellen in Anwesenheit eines Fragmentes von Fibronectin oder von anderen Glykoproteinen erfolgt.

Gegenstand der Erfindung gemäß US 2007 224 174 ist ein Ligand der AxL-Rezeptor- Tyrisinkinase, der eine Differenzierung von Vorläufer- NK Zellen zu reifen NK Zellen veranlasst. Weiterhin beinhaltet die Erfindung die Herstellung von reifen NK- Zellen durch eine Behandlung von hämatopoetischer Stammzellen mit Interleukin 7, Stammzellenfaktoren und Flt3L und somit die Differenzierung in Vorläufer NK- Zellen im ersten Schritt und in einer weiteren Behandlung dieser Vorläufer NK- Zellen mit wenigstens einem AxL- Rezeptor- Tyrosinkinase zur Differenzierung dieser Vorläufer NK- Zellen in reife NK Zellen im zweiten Schritt.

Ein Verfahren für das Differenzieren der Vorläufer Killer- Zelle in reife NK- Zellen, auch um große Menge der reifen NK -Zellen zu erhalten, wird in der KR 100650384 beschrieben, in dem die Vorläufer- NK- Zelle mit einer Axl -Rezeptor-Tyrosinkinase behandelt wird. Die Lösung für die Differenzierung der Vorläufer Killer- Zellen in reife NK -Zellen besteht aus mindestens einem Ligand, der von den bestehenden Antikörper der Gruppe der Axl -Rezeptor-Tyrosinkinase (Axl) ausgewählt wird, insbesondere das Gamma-carboxyllierte Wachstum fördernde spezifische Protein gene6 (Gas6) und Protein S als Ligand des Axl. Das Verfahren umfasst die Schritte:

(a) Differenzierung der hämatopoetische Stammzellen in Vorläufer NK- Zellen durch eine Behandlung mit Interleukin-7, Stammzellenfaktor und Liganden Flt3, und

(b) das Behandeln der Vorläufer NK -Zellen mit mindestens einem Antikörper, der von den bestehenden Antikörper der Gruppe der Axl Rezeptor-Tyrosinkinase (Axl) ausgewählt wird, insbesondere das Gamma-carboxylierte spezifische Protein gene6 (Gas6) und Protein S.

Alle diese Lösungen bewirken eine Differenzierung von Vorläufer-Killer- Zellen zu Natural- Killer- Zellen (NK) und keine für Transplantationszwecke geeignete Expansion von Natural- Killer- Zellen.

Die JP 4299983 beschreibt ein Verfahren zur Züchtung von Lymphikin-aktivierten Killer- Zellen, wobei periphere Blut- Monozyten mit einem Aminosäure- niederer Alkyl- Ester, wie z.B L-Phenylanalin-Methylester zur Dezimierung der Monozyten behandelt und die restlichen Zellen in einem Medium, das Interleukin- 2 enthält, zu einer Zelldichte von $5 \times 10^6$ bis $1 \times 10^7$ Zellen/ml unter Verwendung eines Kunststoff-Bioreaktors, an dessen Wänden adhärierten Lymphokin- aktivierte Killer- Zellen angeordnet sind, kultiviert werden. Die nicht- adhärenten Zellen werden vom kultivierten Produkt entfernt. Für eine starke Vermehrung der adhärierten LAK- Zellen werden diese in einem Medium, das Interleukin-2 und Interleukin-4 enthält, kultiviert. Die Zellen werden dann einem pharmazeutisch zulässigen Träger zugegeben und zusammen mit Interleukin 2 als Heilmittel für Säuger- Tumore injiziert.

In der EP 0289896 wird ein Verfahren zur Herstellung von Lumphokin- aktivierten Killer- Zellen (LAK- Zellen) beschrieben, wobei eine Lymphozyten enthaltende weiße Blutzellfraktion, die durch Standard Leukapherese, Zentrifugal- Leukapherese oder Standardzentrifugation erhalten wird, zur Herstellung der Lymphokin- aktivierten Killerzellen durch Inkubation mit IL-2 behandelt wird. Eine Entfernung der roten Blutzellen und Granulozyten durch Zentrifugierung auf Ficoll ist nicht notwendig.

In der JP 63202378(A), EP 0280054(A1) und DE 3875752 (T2) wird ein verbessertes Verfahren zur Herstellung von Lymphokin aktivierten Killer- Zellen (LAK- Zellen) oder Tumor- infiltrierende- Lymphozyten offenbart, wobei die Verbesserung die Züchtung von Vorläufer- Zellen in einem geschlossenen Behälter erfolgt, der durch ein Copolymer- Film-Material gebildet wird, das eine spezifische $O_2$ -Permeabilität von > $1,8 \times 10^6$ $\mu m^3/m^2$ x s x Pa und eine spezifische Materialdicke von ungefähr 0,04 Millimeter bis ungefähr 0,23 Millimeter hat.

Die JP 63028388(A) und die korrespondierenden Patentschrift EP 0247613(A2) beschreibt einen Prozess für die Behandlung von Lymphokin aktivierten Killer- Zellen (LAK- Zellen), worin periphere mononukleare Blutzellen zur Herstellung einer Population von Zellen, die cytotoxisch für NK-Zellen resistente Tumore sind, kultiviert werden, wobei die peripheren mononuklearen Blutzellen oder die peripheren Blutlymphozyten mit einem Alkylester einer L-Aminosäure oder einem Hydrochloridsalz davon kontaktiert und anschließend kultiviert werden. Die L-Aminosäuren können Alanine, Asparaginsäure, Cysteine, Glutaminsäure, Glutamine, Phenylalanine, Tyrosine, Tryptophan und Valine oder Mischungen davon sein.

Zur Herstellung von Anti-Tumor- Killer- Zellen, die für eine erfolgreiche Behandlung von bösartigen Tumoren, etc. erforderlich sind, wird Vollblut oder eine Suspension von Leukozyten unter spezifischen Bedingungen durch eine Säule geführt, in der eine Packung aus Methyl- Methacrylatdivinylbenzol- Copolymer, beschichtet mit einem statistischen Copolymer -2, insbesondere Hydroxyethylmethacrylat und Diethylaminoethyl- Methacrylat sowie aktiviert mit einem Cyan-Halogenid und einem Lectin der Kermesbeere , angeordnet ist. Das Vollblut oder die Suspension mit Leukozyten wird durch diese Säule mit der Packung aus dem oben beschriebenen Material mit einer linearen Geschwindigkeit von $5 \times 10^{-2}$ cm/sec und einer Verweilzeit von >2min geleitet.

In dieser Säule und mittels des in der JP63028388 (A) beschriebenen Verfahrens werden NK- Zellen aus den mononuklearen Blutzellen oder aus den peripheren Blutlymphozyten selektiert aber nicht expandiert.

In der KR 20070030155(A) wird ein Verfahren für die Herstellung einer großen Menge der reifen natürlichen Killer- Zelle (NK) mit Krebs-abtötender Wirkung aus einer Vorläufer Killer-Zelle (pNK) mittels eines Liganden der Axl Rezeptor-Tyrosinkinase vorgestellt. Das Verfahren zur Erzeugung der Killerzellen umfasst die Schritte:

(a) Behandeln einer hämatopoetischen Stammzelle mit IL-7 (Interleukin 7), SCF (Stammzellenfaktor) und Flt3L (FMS-bezogener Ligand der Tyrosinkinase 3), um die hämatopoetische Stammzelle in eine Vorläufer Killer- Zelle zu differenzieren und

(b) die Behandlung der Vorläufer Killer- Zellen mit dem Liganden der Axl Rezeptor-Tyrosinkinase zur Erzeugung der reifen Killer- Zelle für Behandlung von Krebs, wie Darmkrebs, Brustkrebs, Morbus Hodgkin und akute lymphatische Leukämie.

In der KR 20070030115 (A) wird die Herstellung einer natürlichen Killer- Zelle beschrieben , indem die hämatopoetische Stammzelle mit IL-7 (Interlekin 7), SCF (Stammzellefaktor) und Flt3L (FMS-bezogener Ligand der Tyrosinkinase 3) behandelt wird, um so die hämatopoetische Stammzelle in eine Vorläufer- NK- Zelle zu differenzieren, die dann wiederum mit dem Axl Rezeptor und seinem Liganden Gas6 behandelt wird. Die hämatopoetische Stammzelle wird vom menschlichen Nabelschnurblut isoliert. Das Axl ist ein rekombinantes Axl, das von einer Zelle erzeugt wird, die einen rekombinante Vektor pLXSN-Axl enthält. Die Wirtszelle der Zellen, die das rekombinante Vektor pLXSN-Axl enthalten, sind Makrophagen der Maus RAW264.7. Das rekombinante Gas6 wird von einer Zelle erzeugt, die einen rekombinanten Vektor pcDNA3.1 (+) - GAS6 enthält und die Wirtszelle der Zellen, die den rekombinanten Vektor pcDNA3.1 (+) - GAS6 enthält, ist eine Zellform der Maus OP9.

Auch hier wird wieder die Differenzierung von NK- Zellen aus Vorläufer- Zellen und diese wiederum aus hämatopoetischen Stammzellen und keine Expansion der Killer-Zellen beschrieben.

In der DE 10223536 (A1) wird ein Verfahren zur Trennung nicht-adhärenter Zellen von adhärenten Zellen durch Nutzung der selektiven Adhärenz und der Schwerkraft beschrieben, wobei bei der Trennung das Auftreten von Schwerkräften im Wesentlichen vermieden wird. Das Verfahren kann z. B. mittels einer hängenden (inversen) Zellkultur oder nach dem Prinzip des "hängenden Tropfens" durchgeführt werden. Vorzugsweise dient das erfindungsgemäße Verfahren zur Abtrennung von Monozyten oder NK-Zellen von nicht-adhärenten Lymphozyten, Erythrozyten oder Thrombozyten.

Mittels dieses Verfahrens wird lediglich die NK- Zelle oder Monozyten von nicht adhärenten Lymphozyten, Erythrozyten oder Thrombozyten isoliert und nicht expandiert.

Das Verfahren gemäß WO 2009151182 (A1) betrifft die Kultivierung der selbstaktivierten -Lymphozyten mittels Extrahieren der Lymphozyten aus peripherem Humanblut, wobei in einem ersten Schritt die extrahierten Lymphozyten in einer Kulturflüssigkeit, der IL-2, L-Glutamin und autochthones Plasma, in Anwesenheit anti-CD3, Anti- CD 16 , Antikörper anti-CD56 enthält, suspendiert werden. In einen zweiten Schritt wird die flüssige resultierende Mischkultur des ersten Schrittes in Anwesenheit der Antikörper anti-CD3, anti-CD16 und anti-CD56 einer Kulturflüssigkeit beigemischt, der IL-2, L-Glutamin und autochthones Plasma hinzugefügt sind.

Die Erfindung gemäß DE 3782736 (T2) ist auf ein Verfahren zur Kultivierung von Leukozyten gerichtet, wobei die Leukozyten in Gegenwart eines Lymphokins in einem Perfusionskultursystem, vorzugsweise in einem Extrakapillarraum einer Hohlfaserpatrone gezüchtet werden. Die Leukozyten werden mindestens vier Tage und bis zu 19, vorzugsweise 12 - 16 Tage kultiviert und liefern dabei eine zu erntende Ausbeute von 100 bis 5900 %, bei einer Ausgangszellzahl von 0,5 - 4,4 x $10^7$ Zellen/ml, die in den Extrakapillarraum zur Kultivierung injiziert werden. Die Zellen werden mit rIL- 2 allein oder rIL- 2 und anti-CD 3 monoclonalem Antikörper gezüchtet und mittels normaler Kochsalzlösung, die 1% menschliches Serumalbumin enthält, aus der Hohlfaserpatrone gespült.

Mit diesem Verfahren zur Ernte der Zellen über das Spülen der Hohlfaserpatronen werden die Zellen einem großen Scherstress ausgesetzt und die Vitalität der Zellen wird wesentlich herabgesetzt. Außerdem kann die Expansion der Zellen in diesen Hohlfaserpatronen zur Verengungen bis hin zu Verstopfung der Kapillaren führen und erlaubt auf diese Weise keine homogene und gradientenfreie Kultivierung der Zellen.

In der DE 3788026 werden eine Vorrichtung und ein Verfahren zur Kultivierung von Gewebezellen, insbesondere Lymphokin-aktivierten Killer- Zellen (LAK- Zellen) beschrieben, wobei dieses Gewebezellen zusammen mit einem Kultivierungsmedium und Gas in einem Behälter aus einem semipermeablen, für das Kulturmedium und Gas durchlässigen, Film eingeschlossen, die Gewebezellen während der Kultivierung mit Nährstoffen und Gas durch den semipermeablen Film versorgt und der Behälter während der Kultivierung gedreht oder geschwenkt wird. Die Aussaatmenge betrug $2 \times 10^7$ Zellen/ml und nach einer Kultivierungszeit von 6 Tagen wurde eine Erhöhung der Zellzahl um 1000 Zellen/ml erreicht.

Die FR 1448148 beschreibt ein Verfahren und eine Vorrichtung zum Kultivieren von Gewebezellen, insbesondere LAK - Zellen, bei dem die zu kultivierenden Zellen zusammen mit einem Kulturmedium innerhalb eines aus einem semipermeablen Filmmaterial gefertigten inneren Behälter enthalten sind, welcher innerhalb eines mit Kulturmedium und Gas gefüllten Gehäuses angebracht ist.

Auch die WO 8700548 beschreibt die Kultivierung von Lymphokin- aktivierten Killer-Zellen innerhalb eines aus semipermeablen Wänden bestehenden Behälters, der wiederum in einem äußeren Behälter angeordnet ist, wobei das Kulturmedium in dem äußeren Behälter mittels Umwälzpumpen in Bewegung versetzt wird, um das Kulturmedium homogen zu machen und die Zellen in Dispersion zu halten. Es werden Expansionsraten des 10 bis 60-fachen der Ausgangszellzahl erreicht.

In der DE 102007036611 (A1) wird ein Verfahren und eine Vorrichtung zu Kultivierung lebender Zellen, auch von NK- Zellen in einer Flüssigkeit beschrieben, wobei die Zellen in einen Kulturraum eingebracht werden, der mit einem Reservoir für die Flüssigkeit in Verbindung steht ,wodurch das Volumen der Flüssigkeit im Kulturraum konstant gehalten werde kann. Es muss keine Flüssigkeit oder Medium nachgefüllt oder gewechselt werden, da immer ausreichend Flüssigkeit aus dem Reservoir, das ein größeres Volumen an Kulturmedium als der Kulturraum enthält, in den Kulturraum kontinuierlich zum Volumenausgleich strömen kann. Mit diesem Verfahren und mit dieser Vorrichtung ist eine Kultivierung kleiner Gewebeproben oder geringer Zellzahlen in kleinem Volumen durch eine Verbesserung der Zell- Zell-Kontakte durchzuführen.

Hier wird nur eine Expansion von geringen, für eine Transplantation nicht ausreichenden Mengen an Zellen erreicht.

Die US 5.416.022 beschreibt eine kompakte und leicht montierbare Zellkultivierungsvorrichtung, die mindestens einen Zellkultivierungsabschnitt aufweist, dessen Inneres den Zellkulturraum darstellt. Der Zellkulturraum besteht aus einem Paar von steifen, mit verschiedenen Reliefs versehenen, Platten, die beim Zusammenbau Kavitationen bilden. Im Zellkulturraum ist zwischen der oberen und unteren Membran eine Vielzahl von Hohlfasern angeordnet, wobei die untere und obere Membran miteinander versiegelt sind. Rohre sind mit dem Kulturraum verbunden, über die größere Mengen an $O_2$ zum Zellkulturraum geführt werden.

In der US 4220725, US 4647539 und in der DE 3895414 werden Bioreaktoren beschrieben, die aus Bündeln von semipermablen Kapillaren bestehen.

Allen Hohlfasermodulen oder Bioreaktoren mit semipermeablen Kapillaren/Membranen ist eigen, das während der Kultivierungsphase Strömungswege mit Zellen verstopfen und/oder zuwachsen und somit keine homogene und gradientenfreie Versorgung der Zellen mit Nährstoffen und Gasen ermöglichen. Standisierte, GMP- konforme Prozesse sind damit nicht durchzuführen. In der US 5290700 wird eine Zellkulturvorrichtung beschrieben, bei der die Zellen in Medium in einem Zellkulturraum kultiviert werden. Der Zellkulturraum wird durch mindestens drei semipermeable Membranen gebildet. Eine Membran wird durch eine Platte aus Rohren gebildet und ist für frisches Nährmedium durchlässig. Eine zweite Membran ist aus einer Schicht von Kapillaren geschaffen und ist für gasförmige Medien durchlässig und die dritte Membran dient als ein Mikro-Sieb, das den Durchgang des verbrauchten Nährmediums erlaubt, aber für die Zellen innerhalb des Zellkulturraumes undurchlässig ist.

Die DE 3409501 beschreibt ein Verfahren und Vorrichtung zum Kultivieren von humanen, tierischen pflanzlichen sowie hybriden Zellen und Mikroorganismen, wobei sich die Zellen oder Mikroorganismen in Kammern befinden, die zumindest teilweise durch flächige Membranen begrenzt sind und durch diese Membranen ein Stoffaustausch zur Ver- und Entsorgung der Zellen mit gelösten bzw. gasförmigen Nährstoffen bzw. Stoffwechselprodukten erfolgt, wobei durch die Membranen die Zellen im Wesentlichen zurückgehalten werden. Die Kultivierung erfolgt in einem Bioreaktor mit wenigstens drei aneinander liegenden Kammern, wobei wenigstens eine erste Kammer eine Zellkultivierungskammer, eine zweite Kammer eine Medienkammer und eine dritte Kammer eine Produktkammer ist und die einzelnen Kammern einander durch Membranen begrenzt sind, wobei eine Nährlösung enthaltene Medienkammer in ihrer Längsrichtung durchströmt wird und die Zellkultivierungskammer sowie die daran anschließende Produktkammer mit ihrem Inhalt sich in relativer Ruhe zur Strömungsgeschwindigkeit der Nährlösung befinden. Die Vorrichtung besteht aus wenigstens einer Zellkultivierungskammer, die über eine Membran mit der Produktkammer und über eine weitere Membran mit der Medienkammer in Verbindung stehen, wobei die Membranen unter schiedliche Porengrößen aufweisen.

Die DE 4229334 beschreibt ein rollflaschenähnliches Kulturgefäß für Zellkulturen mit einer Zelldichte von mindestens $10^7$ Zellen/ml, das, eine Versorgungskammer bildend, ein Nährmedium aufnimmt, in dem mindestens eine, die Zellkulturen aufnehmende Produktionskammer angeordnet ist, die von dem Nährmedium durch eine Dialysemembran, durch die von der Versorgungskammer Nährstoffe in die Produktionskammer und von dort Stoffwechselprodukte in die Versorgungskammer transportiert werden, getrennt ist, und mit einer Gas-Zu- und Abführung in die Versorgungskammer, wobei als Gas-Zu- und Abführung eine für Flüssigkeiten und die Zellkulturen kontaminierende Keime undurchlässige und für die bei der Zellkultivierung benötigten und entstehenden Gase durchlässige Gasaustauschmembran vorgesehen ist, die mindestens einen Teil der Außenwand des Kulturgefäßes bildet und deren Gesamtfläche, Material und Dicke so gewählt sind, dass die Durchlässigkeit für Sauerstoff pro $10^7$ Zellen mindestens 0,01 mg/h beträgt.

Das Verfahren beschreibt eine Zellkultivierung in einem rollflaschenähnliches Kulturgefäss,das mit einem Nährmedium gefüllt ist und das eine allseitig geschlossene und die zu expandierende Zellkultur aufnehmende Produktionskammer umspült. Das Kulturgefäß wird in eine kontinuierliche Rollbewegung versetzt wird, wobei die Produktionskammer derart mit der zu kultivierenden Zellkultur gefüllt wird, dass sie eine Luftblase enthält, deren Volumen mindestens 10% des Volumens der Produktionskammer einnimmt, und dass der Rollbewegung eine Taumelbewegung des Kulturgefäßes überlagert wird, derart, dass die Luftblase infolge des Auftriebs in der Zellkultur bewegt wird. Durch die Trommelbewegung wird zwar eine dynamische Versorgung der Zellen erreicht aber eine homogen, gleichbleibende Mengen an Zellen kann mittels dieses Verfahren nicht kultiviert werden, da sich das Volumen des Kulturmediums durch die Taumelbewegung ständig verändert. Ein homogenes, gleichbleibendes Verhältnis Zellmenge zur Kultivierungsfläche bzw. Volumen sowie eine lockere Packung der Zellen untereinander wird zwar erreicht, aber durch die Taumelbewegung werden diese Kontakte ständig unterbrochen und dichtere Schichten von Zellen können mit diesem Verfahren nicht hochwachsen. Außerdem sind die Zellen während des Kultivierungsvorganges durch die Taumelbewegung einem ständigem Scherstress ausgesetzt, der einen negativen Einfluss auf die Vitalität der Zellen ausübt.

Die WO 9318133 beschreibt eine Vorrichtung für die Behandlung von Zellkulturen auf plattenförmigen Trägern, die mindestens teilweise gasdurchlässig sind und auf denen eine Kollagenbeschichtung mit der zu kultivierenden Zellkultur aufgetragen wird. Diese Kollagenbeschichtung mit der zu kultivierenden Zellkultur ist sehr kompliziert und bei medizinischer Anwendung ungünstig und zudem teuer.

Die DE 602004008453(T2), EP 1625212(B1) und insbesondere die EP 1479760(A1) offenbaren ein Verfahren und eine Vorrichtung zur Kultivierung von Zellen und/oder Gewebe, wobei die Kultivierung von in einem oder mehreren Kulturräumen immobilisierten Zellen unter Kontakt mit wenigstens einer zellfreien Versorgungsflüssigkeit und wenigstens einer mobilen Phase , die Zellen eines anderen Zelltyps bezogen auf die immobilisierten Zellen enthalten, ausgeht.

Die Kulturräume weisen eine Matrix auf, die vorzugsweise aus Gelen, poröse Materialien, wie offenporigen Schäumen usw., Geweben, Vliesen usw.besteht.

Die immobilisierten Zellen sind Säugerzellen, insbesondere humane Zellen, wobei hämatopoetische Stammzellen, dendritische Zellen, Monocyten, Makrophagen oder andere primäre oder transformierte Zellen besonders bevorzugt eingesetzt werden. Die zellfreie Versorgungsflüssigkeit ist eine für die Zellen und/oder das Gewebe angepasste Nährlösung. Die Zellen der mobilen Phase sind Zellen mit regulatorischen oder später antikörperbildenden Eigenschaften und insbesondere mononukleare Zellen, wie T- und B- Lymphozyten usw. Die zellfreie Versorgungsflüssigkeit und die mobile Phase sind kontinuierlich austauschend ,wobei die zellfreie Versorgungsflüssigkeit weiterhin zum Abtransport der von dem Gewebe und/oder den Zellen gebildeten Metaboliten/Zellprodukte dient und wobei die zellfreie Versorgungsflüssigkeit und die mobile Phase eine unterschiedliche Strömungsgeschwindigkeit aufweisen.

Die Vorrichtung zum Kultivieren von Zellen und/oder Gewebe, umfasst wenigstens einen Kulturraum zum Ansiedeln von Zellen und/oder Gewebe, dem unterschiedliche Flüssigkeitsströme, wenigstens eine zellfreie Versorgungsflüssigkeit und wenigstens eine mobile Zellphase , kontinuierlich zuführbar sind. Die Vorrichtung enthält im Kulturraum eine Matrix und die Flüssigkeitsströme strömen oder diffundieren an für Zellen permeablen Kontaktflächen entlang der Umfangsflächen des Kulturraums oder der Matrix oder durch die Matrix hindurch, wobei die Flüssigkeitsströme dem Kulturraum oder der Matrix über permeable Leitungen zu geführt werden und wenigstens eine der Leitungen für die mobile Zellphase permeabel ist und

wenigstens eine Versorgungsflüssigkeit über permeable Leitungen, insbesondere Kapillaren usw., durch oder entlang des Kulturraums oder der Matrix zuführbar sind. Die Immobilisierung der Zellen in den Vliesen verursacht nicht zu kontrollierende Gradienten und somit ist eine gradientenfreie Versorgung der Zellen und/oder des Gewebes nicht möglich. Weiterhin ist mit dieser Lösung das Verhältnis Zellmenge zur Kultivierungsfläche nicht optimierbar und es kann kein physiologisches Verhältnis von Zellenzahl zur Fläche eingestellt werden.

_In der FR 2608169 wird ein Modul zur Kultivierung für die Züchtung von Mikroorganismen beschrieben , das aus einen im wesentlichen flachen Behälter der durch Trennwände in mehrere Kanäle unterteilt ist, die untereinander durch einen Serpentinenkanal verbunden sind, besteht.

Mit diesem Modul stellt kein mäanderartiges Modul dar und kann im Verlauf der Kultivierung der Zellen im Modul kein laminarer Mediumfluss erzeugt werden, weil dafür entsprechende Zu- und Ablaufanschlüsse fehlen und die Trennwände untereinander durch einen Serpentinenkanal verbunden sind.

In dem Journal Immunol Methods, 2009 June 30; 345 (1-2):90-99 wird ein geschlossenen Ein- Durchgangs- System für eine schnelle Expansion von Lymphozytenkulturen für die Adoptiv- Zell- Therapie beschrieben. Der Bioreaktor von Aastrom Bioscience Inc. (Ann Arbor, MI) mit einem geschlossenen System ist eine einfache Zirkulationskammer, die aus einem Zellbett-, einem Gas- und einem Medienabfallbereich besteht, wobei auf dem Grund der Zellbettkammer, die ein Fassungsvermögen von 300 ml hat, Zellkulturträger mit einer Gesamtfläche von 850 cm$^2$ angeordnet sind. Die Nährstoffflüssigkeit wird über einen zentralen Eingangsport über die zu kultivierenden Zellen geführt und die verbrauchte Nährstoffflüssigkeit mit den Abfallprodukten über einen Ausgangsport abgeführt. Die Flüssigkeitskammer mit dem Zellbettbereich ist durch eine semipermeable Membran, die für die Gase durchlässig und für die Nährstoffflüssigkeit, Kulturflüssigkeit und Zellen undurchlässig ist, getrennt. Die Gase, wie $O_2$, $N_2$, $CO_2$ werden über einen Gaszuführungsport eingeführt, über die Membran zum Ausgangsport geleitet, wobei die Gase durch die semipermabele Membran in die Nährstoffflüssigkeit diffundieren. Die Entkopplung des Gastransportes von der Medienperfusion erlaubt einen langsamen laminaren Fluss des Mediums über die Zellen, so dass dieser Bioreaktor auch für die Kultivierung nichtadhärenter Zellen (Lymphozyten) eingesetzt werden kann. Mit diesem Bioreaktor sind Expansionsraten beim antigenspezifischen TIL (Tumor infiltrating Lymphocytes) von 1: 1127 möglich. Der Einsatz der semipermeablen Membranen und die Entkopplung des Gastransportes von der Mediumperfusion ermöglichen, den für die Expansion notwendige Kontakt der Zellen untereinander und eine gradientfreie, homogene Versorgung der Zellen ist zumindest zu Beginn der Kultivierung mit dieser Lösung gegeben. Mit zunehmender Zeit der Kultivierung besteht aber die Möglichkeit, dass sich Zellen an der semipermeablen Membran ansiedeln und so die homogene Versorgung der Zellen verhindern. Außerdem kann keine zusammenhängende, für eine hohe Expansionsrate notwendige Zellschicht aufgebaut und kein auf ein optimiertes Zellwachstum ausgerichtetes Verhältnis Zellenzahl zur Fläche eingestellt werden, da die Zellen auf mehreren Zellkulturträgern adhärieren und dort expandieren. Die Zellen müssen von diesen Zellkulturträgern mittels Enzymen oder Zellschaber geerntet werden, was die Zellen beschädigt. Außerdem ist der Aufbau dieses Bioreaktors durch den Einsatz von semipermablen Membranen kompliziert und für den medizinischen Alltag unbrauchbar.

Alle bekannten Verfahren für die Vermehrung von hämatopoetischen Zellen/Stammzellen und die in den Verfahren benutzten Bioreaktoren oder Vorrichtungen sind dadurch gekennzeichnet, dass die Kultivierungsbedingungen kompliziert sind, in der Mehrzahl der Verfahren ein erheblicher apparativer Aufwand erforderlich ist und bei wichtigen Zellen (z. B. NK-Zellen, stimulierten spezifischen T-Lymphozyten) die Expansion nicht zu therapeutisch erwünschten Mengen führt. Vielfach werden Zellen in Zellkulturkammern vermehrt, die mittels semipermeabler Membranen/Hohlfasern von Nährmedium- und/oder Gasversorgung getrennt sind. Diese komplexen Systeme sind fehleranfällig, für den Einsatz im klinischen Alltag wenig geeignet und für den Betrieb unter GMP-Bedingungen oft untauglich.

Der Erfindung liegt die Aufgabe zugrunde, einen Bioreaktor und ein Verfahren zu entwickeln, die es ermöglichen, ausreichende Mengen an hämatopoetischen Zellen/Stammzellen/Progenitorzellen für therapeutische Anwendungen und Transplantationszwecke herzustellen. Der Bioreaktor soll einfach aufgebaut, als Disposable-Version herzustellen und, das Verfahren für die Zellexpansion,-Differenzierung, -Stimulierung und -Ernte soll sicher, einfach und transparent durchzuführen sein, um alle Vorgaben für Zellpräparate hinsichtlich GMP-Konformität und Erlangung von Herstellungserlaubnissen für die im Bioreaktor hergestellten hämatopoetische Zellpräparate zu erfüllen. Weiter soll der Bioreaktor im klinischen Alltagsbetrieb ohne großen Aufwand einzusetzen sein.

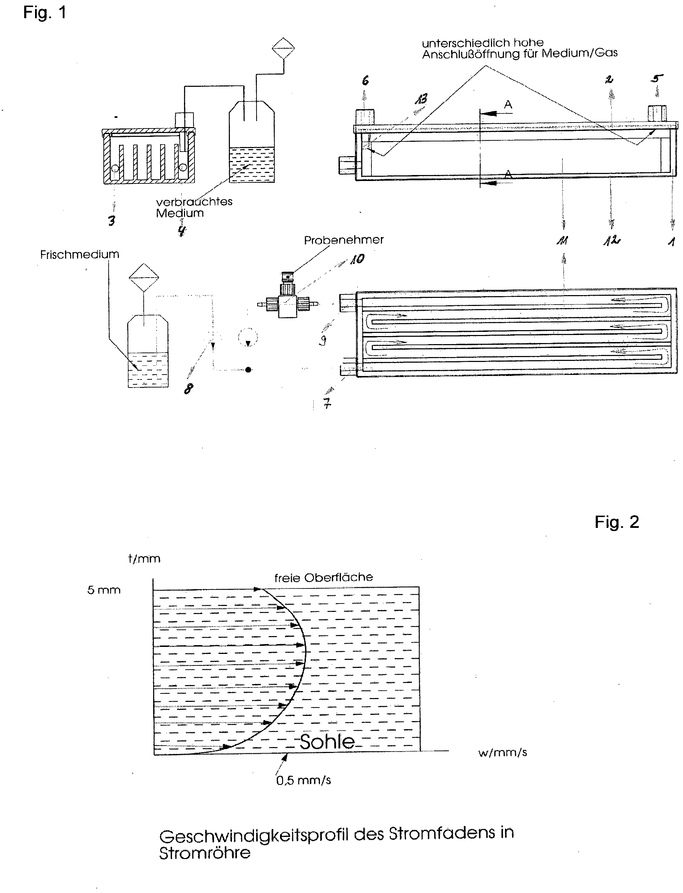

Erfindungsgemäß wird die Aufgabe durch einen Mäander-Bioreaktor gelöst, der schematisch in Fig. 1 dargestellt ist. Der Bioreaktor besteht aus einem rechteckigen Bioreaktorgefäß (1) aus klarem Polymermaterial besteht. Das Gefäß ist mit einem Deckel (2) steril verschlossen oder besitzt in der Disposable-Version einen geschlossenen Innenraum. Grundfläche des Bioreaktors kann zwischen 20 und 500 cm$^2$ betragen, bevorzugt zwischen 50 und 100 cm$^2$, die Höhe des Bioreaktors beträgt zwischen 1,8 und 6 cm, bevorzugt 2,5 cm. Der Bioreaktor ist mit Anschlussöffnungen (3, 4, 5, 6) versehen, die unterschiedlich hoch vom Boden angebracht sind und durch die das Gefäß mit Medium und mit Gasen durchströmt wird. An der Anschlussöffnung (3) des Medienkreislaufes ist eine Zuführung (7) mit einer zusätzlichen Zuführung (8) für Frischmedium und an der Anschlussöffnung (4) des Medienkreislaufes ist eine Abführung (9) mit einem Zugang (10) für eine sterile Probeentnahme angeordnet.

Entscheidendes Merkmal des Bioreaktors ist die Aufteilung des Innenraumes dergestalt, dass dünne Trennwände (11) die gesamte Bodenfläche (12) des Bioreaktorgefäßes (1) streifenförmig unterteilen. Sie sind so angeordnet, dass perfundiertes Medium zu einer mäanderförmigen Durchströmung des gesamten Bioreaktorgefäßes (1) gezwungen wird. Die Trennwände (11) können eine Höhe von 30% bis 80% der Gesamthöhe des Bioreaktorgefäßes haben, bevorzugt 60% bis 75%. Oberhalb der Trennwände (11) ist der Innenraum nicht unterteilt und dient zur Überströmung mit Gasen. Der Abstand (A) der Trennwände (11) voneinander sowie von den Seiten- und Stirnwänden des Bioreaktorgefäßes (1) und die Wandhöhe der Trennwände (8) wird so gewählt, dass sich in den Stromfäden, in den durch die Trennwände (8) gebildeten Gerinne, eine gemittelte laminare Oberströmung mit einer Froudezahl <0,005, vorzugsweise 0,002 und eine Bodenströmung mit einer Froudezahl von 0 oder nahe 0 bildet, wobei die Froude- Zahl aus den Größen berechnet wird, die in der nachfolgenden Formel aufgeführt sind:

$$Fr = \frac{w}{\sqrt{g*t}}$$

dabei ist: w die gemittelte Fließgeschwindigkeit in m/s

g die Erdbeschleunigung in m/s$^2$

t die Gerinnetiefe in m

Der Abstand (A) der Trennwände (11) voneinander sowie zu den Seiten- und Stirnwänden des Bioreaktorgefäßes (1) kann 2 bis 10 mm, bevorzugt 4 bis 5 mm betragen.

Durch die Kompartimentierung der Bodenfläche des Bioreaktorgefäßes konnte überraschenderweise erreicht werden, dass über die gesamte Fläche eines Bioreaktors eine laminare Oberschicht aus umlaufenden Medium und eine weitgehend ruhende Medium-Unterschicht aufrecht erhalten wird, wobei die Medienzusammensetzung über die gesamte Fläche sehr weit gehend homogen ist und stabile Gradienten zwischen Ober- und Unterschicht sich ausbilden. Zusätzliche Beschichtungen, Gele u.ä. für die Immobilität der Zellen sind dabei nicht nötig.

Die pro Zeit durch das beschriebene Bioreaktorgefäß (1) perfundierte Medienmenge bestimmt (neben der Raue der Materialoberflächen und der Viskosität des Mediums) bei gegebenen Abmessungen des Bioreaktors und Abständen (A) der Trennwände (11) die Fließgeschwindigkeit des Mediums. Die Fließgeschwindigkeit kann in einem weiten Bereich durch entsprechende Einstellung der Medium-Perfusionsrate gewählt werden. Sie kann so eingestellt werden, dass die obere Schicht des umlaufenden Mediums eine laminare Strömung ausbildet, während nahe am Boden und an den Wänden des Bioreaktorgefäßes (1) die Strömung zum Stillstand kommt. Einlauf- und Auslauföffnung (3, 4) für das Medium sind entsprechend angeordnet. Zwischen der laminar umlaufenden oberen Medienschicht und dem am Bioreaktorboden oder nahe dabei befindlichen Medium bestehen Gradienten bezüglich der Konzentrationen an Nährstoffen und Gasen. Diese Gradienten sind aber über den ganzen Stromfaden weitgehend identisch. Wenn hohe Zelldichten im Bioreaktorgefäß (1) erreicht sind, werden größere Mediummengen pro Zeit benötigt. Inhomogenitäten und Störungen des Strömungsprofiles werden dann dadurch vermieden, dass das überstehende Volumen durch Höherstellung der verstellbaren Anschlussöffnung (6) mit Überlauf (13) vergrößert und/oder auch der Zufluss von Frischmedium gesteigert und der Mediumumlauf beschleunigt werden.

Der vorstehend beschrieben Typ eines Mäander-Bioreaktors ist für die Expansion verschiedener hämatopoetischer Zellen/Stammzellen/Progenitorzellen bestens geeignet. Generell ist das Kultivierungs-Verfahren im Mäander-Bioreaktor auf folgende Weise durchzuführen: Die Zellen werden in Medium suspendiert, die Suspension wird in den Bioreaktor eingefüllt. Die Menge an Medium wird so gewählt, dass die unteren Ein- und Ausgänge für den Umlauf mit Medium bedeckt sind. Der Bioreaktor bleibt unbewegt im GMP Breeder der Z® RP Zellkultivierungsanlage stehen. Die Zellen setzen sich in 60 bis 120 min auf dem Boden des Bioreaktors ab. Die jeweils notwendige Ansaatmenge an Zellen richtet sich nach der Zellart. Wenn die Gesamtfraktion der Mononuklearzellen aus peripherem humanem Blut, aus Nabelschnurblut oder aus Knochenmark zu vermehren ist, kann die Zellzahl für die Besiedlung klein gehalten werden (etwa 0,1 bis 0,2 x 10$^6$ Zellen pro cm$^2$). Eine gute Expansion isolierter Fraktionen hämatopoetischer Zellen (z. B. NK-Zellen, T-Lymphozyten) setzt voraus, dass die entsprechenden Zellen nach dem Sedimentieren eine ruhende Zellschicht bilden, in der sie sich berühren, wobei bei diesen Zellen Zellzahlen von von 0,1 bis 2 x 10$^6$ Zellen/cm$^2$, vorzugsweise 0,5 bis 1x 10$^6$ Zellen/cm$^2$ erforderlich sind.

Für die Expansionsphase wird der Kultivierungsprozess durch Umpumpen von Medium, Überströmung mit Gasen, kontinuierliche Regelung von pH und pO$_2$ in Gang gesetzt. In täglichen Probennahmen von umlaufendem Medium werden Glukoseverbrauch und Laktatbildung bestimmt. Die sedimentierten hämatopoetischen Zellen werden laminar so mit Medium überströmt, dass keine Zellen aufgewirbelt werden.

Das nach der Bildung der Zellschicht zellfreie Kulturmedium wird zunächst mit Raten von 1- 20 x/h umgepumpt und dabei werden über den sterilen Anschluss (10) regelmäßig, vorzugsweise 1x täglich Proben genommen. Bei Absinken des Glukosegehaltes auf 1/3 des Ausgangswertes im Kulturmedium wird über die Zuführung (8) für Frischmedium kontinuierlich steigende Mengen an frischem Medium zuführt, wobei die Zufuhr von frischem Medium so eingeregelt wird, dass der Glukosegehalt >1/3 des Ausgangsgehaltes sowie der Laktatgehalt < 3g/l im Kulturmedium beträgt und die Medienmenge im Bioreaktor nur um den Faktor 3 gegenüber der Menge der Zellsuspension beim Start der Kultivierung ansteigt. Erfordert der Glukosegehalt eine höhere Zufuhr von Frischmedium, wird das verbrauchte Kulturmedium über den Überlauf (13) abgeführt. Das Verhältnis der zirkulierenden Medienmenge zur zugeführten Menge an Frischmedium soll dabei 5/1 nicht überschreiten.

Empfehlenswert ist, den Bioreaktor täglich mit einem Durchlicht-Mikroskop zu inspizieren, das vor dem GMP Breeder aufgestellt wird. Damit kann der Fortgang der Kultivierung beobachtet und bewertet werden.

Die Gesamtfraktionen von Mononuklearzellen vermehren sich bereits nach 1 bis 2 Tagen so intensiv, dass eine dicke Bodenschicht aus Zellen ausgebildet wird, auf die zusätzlich große Zellklumpen aufgelagert sind. Durch kurzes kräftiges Aufschütteln der Zellen und anschließende Ruhephase von 60 min ohne Umpumpen werden die Zellen erneut gleichmäßig auf dem gesamten Bioreaktorboden verteilt. Die Expansion kann dann fortgesetzt werden. In 10 bis 15 Tagen können die Mononuklearzellen mindestens um den Faktor 1000 expandiert werden. NK Zellen, T-Lymphozyten, B-Lymphozyten, Monozyten zeigen ebenfalls gute Vermehrungsraten, wenn sie, verglichen mit Mononuklearzellen, anfangs in solchen Mengen pro Bioreaktorfläche angesiedelt werden, dass sie nach dem Absetzen am Boden eine Schicht bilden, in der sich viele Zellen berühren. Bei reinen Fraktionen der vorstehenden Zellarten werden in 10 bis 25 Tagen Kultivierungsdauer Vermehrungsraten um den Faktor 20 bis 200 erreicht.

Durch die nachfolgen Ausführungsbeispiele wird die Erfindung näher erläutert, wobei in der Fig. 1 eine schematische Darstellung des Bioreaktors beschrieben und in der Fig. 2 der Strömungsverlauf über die Gerinnetiefe dargestellt wird, wobei

1     Reaktorgefäß
2     Deckel
3     Anschlussöffnung für die Medienzuführung
4     Anschlussöffnung für die Medienabführung
5     Anschlussöffnung für die Gaszuführung
6     Anschlussöffnung für die Gasabführung
7     Zuführung Medienkreislauf
8     Zuführung für Frischmedium
9     Abführung Medienkreislauf
10    Steriler Zugang für Probeentnahme
11    Trennwände
12    Bodenfläche
13    Überlauf für verbrauchtes Kulturmedium
A     Abstand der Trennwände untereinander

bedeuten.

Ausführungsbeispiel 1:

Natural Killer-Zellen (NKs) werden nach einem üblichen, in der Literatur beschriebenen Protokoll aus peripherem Human-Blut in reiner Form isoliert. Die Zellen werden in SCGM-Medium aufgenommen, dem 10 % Humanserum und IL2 als Supplemente zugesetzt sind.

Ein Bioreaktorgefäß mit 50 ml Volumen wird im GMP Breeder der Zellkultivierungsanlage aufgestellt und über Luer Lock-Verbindungen an Medium- und Gasversorgung sowie Control Unit mit Pumpen angeschlossen. Der Bioreaktor ist eine steril verpackte Einheit mit integrierten Sondengefäßen für pH- und $pO_2$-Sonde, versehen mit Schlauchverbindungen für die Medium-Zirkulation über die Zu- und Abführung für Medien (7,9), die Zuführung (8) von Frischmedium und Überlauf der Zellbrühe (13), die Zu- und Abfuhr von Gasen (6,7) sowie einem Anschluss (10) für die Probenentnahme.

Die Suspension der NK-Zellen wird über die Zuführung für Medien(7) in das Bioreaktorgefäß eingefüllt. Medium-Zirkulation und Gaszufuhr sind ausgeschaltet. Durch kurzes Schwenken wird die Suspension gleichmäßig im Bioreaktorgefäß verteilt. Die Ansaatmenge soll 0,5 bis 1x10^6 Zellen/ cm$^2$ Grundfläche des Bioreaktorgefäßes betragen. Der Bioreaktor wird sodann wagerecht ausgerichtet im GMP Breeder der Bioreaktoranlage aufgestellt, bei 37 °C inkubiert, die Gaszufuhr wird eingeschaltet. In 60 bis 120 min setzen sich die Zellen als lockere Schicht auf dem Boden des Bioreaktors ab. Nach diesem Zeitraum wird die laminare Überströmung der Zellschicht mit Medium in Gang gesetzt.

Zunächst wird das von Zellen freie Medium im Bioreaktor über die Zu- und Abführungen für Medien (7,9) nur umgepumpt. Die Sonden für die Messung von pH und $pO_2$ sind in den Kreislauf eingebunden, die vorgewählten pH- und $pO_2$-Werte werden geregelt. Die Strömungsgeschwindigkeit des Mediums wird mit der Control Unit der Bioreaktoranlage so eingestellt, dass die Froude-Zahl 0,002 beträgt und die abgesetzten Zellen nicht aufgewirbelt werden. Letzteres kann im klar durchsichtigen Bioreaktorgefäß gut beobachtet werden. Über den im Mediumkreislauf befindlichen sterilen Zugang (10) für die Probeentnahme werden regelmäßig (z. B. 1 x täglich) Medienproben entnommen. In den Proben werden Glukoseverbrauch und Laktatbildung im jeweiligen Zeitraum gemessen. Wenn der Glukosegehalt auf etwa 1/3 des Ausgangswertes gesunken ist, werden über die Zufuhr für Frischmedium (8) dem umgepumpten Medium kontinuierlich steigende Mengen an frischem Medium zugesetzt. Die Zufuhr von Frischmedium wird so geregelt, dass der Glukosegehalt im umlaufenden Medium nicht unter 1/3 des Ausgangsgehaltes fällt und der Laktatgehalt 3 g/l nicht überschreitet. Die Medienmenge im Bioreaktor wird dabei um den Faktor 3 größer, verglichen mit der Zellsuspension beim Start der Kultivierung. Wenn der Glukoseverbrauch eine darüber hinausgehende Zufuhr von Frischmedium nötig macht, wird die verbrauchte Zellbrühe über den Überlauf für die Zellbrühe (13) durch den Gasstrom in ein entsprechendes Gefäß gedrückt. Das Verhältnis von zirkulierender Mediummenge und zugeführter Menge an Frischmedium soll 5 zu 1 nicht überschreiten. Insgesamt ist auf diese Weise eine Vermehrung der Ansaat-Zellen auf etwa die 50-fache Zahl an NK-Zellen möglich.

Um die Zellen zu ernten, wird das Bioreaktorgefäß kurz kräftig geschüttelt. Vor dem Schütteln wird die Gaszufuhr geschlossen und die Zirkulation des Mediums abgestellt. Die Suspension von NK-Zellen wird dann über die Abführung der Zellbrühe (6) für die weitere Verwendung in vorbereitete Gefäße verbracht (z.B. Zentrifugenröhrchen).

Eine weitere Expansion der NK-Zellen ist machbar. In diesem Fall wird die Zellsuspension aus dem Bioreaktorgefäß nicht geerntet, sondern über die Abführung (13) für die Zellbrühe in ein zweites Bioreaktorgefäß mit entsprechend größerer Fläche überführt. Die weitere Kultivierung wird analog durchgeführt wie vorstehend für das erste Bioreaktorgefäß beschrieben. Eine Vermehrung der NK-Zellen auf das 200 bis 500-fache der Ansaat-Zellmenge wird mit diesem Verfahren erreicht.

Das Wachstum der NK-Zellen kann ohne weiteres mikroskopisch verfolgt werden. Dazu wird der (steriltechnisch geschlossene) Bioreaktor auf ein vor dem GMP-Breeder aufgestelltes Durchlicht-Mikroskop gestellt. Die Zellen liegen zu Beginn der Expansion als dünne Schicht homogen verteilter, gleichmäßig geformter NK-Zellen auf dem Boden des Bioreaktors. Nach wenigen Tagen bilden sie einen dicken Bodenbelag aus gleichmäßig geformten NK-Zellen. Bei weiterer Vermehrung der Zellen bilden sich Klumpen von NK-Zellen aus, die über die ganze Oberfläche des Bioreaktorbettes verteilt sind. Wenn der Bioreaktor kurz kräftig geschüttelt wird, zerfallen die Zellklumpen, es entsteht eine homogene Zellsuspension. Die Zellen dieser Suspension setzen sich in 60 bis 120 min erneut auf dem Bioreaktorboden ab.

**Patentansprüche**

1. Mäander- Bioreaktor zur dynamischen Expansion, Differenzierung und Ernte von nicht adhärent wachsenden Zellen, insbesondere von Gemischen oder isolierten Fraktionen hämatopoetischer Zellen, Stammzellen und Progenitorzellen humanen Ursprungs, **dadurch gekennzeichnet, dass** der Mäander- Bioreaktor aus einen rechteckigen Bioreaktorgefäß (1) aus vorzugsweise klarem Polymermaterial besteht, der mit einem Deckel (2) steril verschlossen ist und an dem in unterschiedlicher Höhe angebrachte Zu- und Abführungen (3, 4, 5, 6) für die Durchströmung mit Medium und Gasen angeordnet sind, wobei die Bodenfläche (12) des Bioreaktorgefäßes (1) 20 bis 500 cm$^2$, vorzugsweise 50 bis 100 cm$^2$ beträgt und die Bodenfläche (12) mit streifenförmigen , die Bodenfläche (12) unterteilenden und eine mäanderförmige Durchströmung des Bioreaktorgefäßes (1) mit Gasen und Medium erzwingenden Trennwände (11) versehen ist, wobei der Abstand (A) der Trennwände (11) voneinander sowie zu den Seiten- und Stirnwänden des Bioreaktorgefäßes (1) 2 bis 10 mm, bevorzugt 4 bis 5 mm beträgt_und sich in den Stromfäden, in den durch die Trennwände (8) gebildeten Gerinne, eine gemittelte laminare Oberströmung mit einer Froudezahl < 0,005, vorzugsweise 0,002 und eine Bodenströmung mit einer Froudezahl von 0 oder nahe 0 bildet.

2. Mäander- Bioreaktor nach Anspruch 1, **dadurch gekennzeichnet, dass** der an der Anschlussöffnung (6) angeordnete Überlauf (13) für die verbrauchte Zellbrühe in seiner Höhe verstellbar angeordnet ist.

3. Mäander- Bioreaktor nach Anspruch 1-2, **dadurch gekennzeichnet, dass** über den Überlauf (13) für die Zellbrühe (mit nicht näher dargestellten Absperrvorrichtungen) mehrere, insbesondere 2 bis 5 Biofilmreaktoren in Reihe modulartig miteinander verbunden sind, wobei das nachgeschaltete Modul eine um die Expansionsrate von 1:10 bis 1:50 größere Bodenfläche (12) des Bioreaktorgehäuses (1) gegenüber der Bodenfläche (12) des Bioreaktorgehäuses (1) des vorgeschalteten Modul aufweist.

4. Verfahren zur dynamischen Differenzierung und Ernten von hämatopoetischen Zellen, insbesondere von Natural- Killer- Zellen (NK- Zellen), T-Lymphozyten und B- Lymphozyten nach Anspruch 1, außer humanen embryonalen Stammzellen, **dadurch gekennzeichnet, dass** eine die Zuführung (7) und die Abführung (9) für den Medienkreislauf bedeckende Menge in Medium suspendierter Zellen über die Zuführung (7) dem waagerecht aufgestelltem Bioreaktorgefäß (1) in einer, eine vollständige Bedeckung der Bodenfläche (12) des Bioreaktorgefäßes (1) und in einer, eine Zellschicht mit einem engen Kontakt der Zellen untereinander erzwingenden Zellzahl von 0,1 bis 2x10$^6$, vorzugsweise 0,5 bis 1x10$^6$ Zellen/cm$^2$ zugeführt, das Bioreaktorgefäß (1) über einen Zeitraum von 30 bis 180, vorzugsweise 60 bis 120 min in einer waagerechten Lage und in Ruhe gelagert, danach die Gaszufuhr über die Zuführung (5) und Abführung (6) mit einer, eine laminare und keine Verwirbelung erzeugende Strömung eingeregelten Gasmenge zugeschaltet, das zellfreie Kulturmedium mit Raten von 1 bis 20 x/h, vorzugsweise 5 bis 15 x/h umgepumpt wird und dabei über den sterilen Zugang (10) regelmäßig Proben entnommen werden, wobei bei Absinken des Glukosegehaltes auf 1/3 des Ausgangswertes im Kulturmedium über die Zuführung (8) Frischmedium mit kontinuierlich steigender Menge zugeführt wird, so dass der Glukosegehalt > 1/3 des Ausgangswertes sowie der Laktatgehalt < 3g/l beträgt, die Medienmenge im Bioreaktor nicht über einen Faktor 3 gegenüber der Menge der Zellsuspension zum Start der Kultivierung ansteigt und das Verhältnis der zirkulierenden Medienmenge zur zugeführten Menge an Frischmedium von 5 zu 1 nicht überschreitet.

**Claims**

1. Meander bioreactor for dynamic expansion, differentiation and harvesting of non-adherent growing cells, In particular of mixtures or isolated fractions of hematopoietic cells, stem cells and progenitor cells of human origin, **characterized in that** the meander bioreactor preferably consists of a rectangular bioreactor vessel (1) there is clear polymer material, which is sterile closed with a lid (2) and on which inlets and outlets (3, 4, 5, 6) are arranged at different heights for the flow of medium and gases, the bottom surface (12) of the bioreactor vessel (1) is 20 to 500 cm$^2$, preferably 50 to 100 cm$^2$, and the bottom surface (12) is provided with strip-shaped partition walls (11) which divide the bottom surface (12) and meander through the bioreactor vessel (1) to force gases and medium is, the distance (A) of the partitions (11) from each other and to the sides d end walls of the bioreactor vessel (1) Is 2 to 10 mm, preferably 4 to 5 mm and there is an average laminar upstream flow with a Froude number <0.005, preferably 0,002 and a bottom flow in the flow threads, in the channels formed by the partition walls (8) with a Froude number of 0 or close to 0.

2. Meander bioreactor according to claim 1-2, **characterized in that** the overflow (13) arranged at the connection opening (6) is arranged adjustable in height for the spent cell broth.

3. meander bioreactor according to claim 1-3, **characterized in that** via the overflow (13) for the cell broth (with shut-off devices, not shown), several, in particular 2 to 5, biofilm reactors are connected in series In a modular manner, the downstream module being one by the expansion rate of 1:10 to 1:50 larger floor area (12) of the bioreactor housing (1) compared to the floor area (12) of the bioreactor housing (1) of the upstream module.

4. A method for the dynamic differentiation and harvesting of hematopoietic cells, in particular natural killer cells (NK cells), T-lymphocytes and B-lymphocytes according to claim 1, except for human embryonic stem cells, **characterized In that** the feed (7) and the discharge (9) for the medium circuit covering the quantity of cells suspended in medium via the feed (7) to the horizontally positioned bioreactor vessel (1) In one, a complete covering of the bottom surface (12) of the bioreactor vessel (1) and in one Cell layer with a close contact of the cells to each other enforcing cell number of 0.1 to $2\times10^6$, preferably 0.5 to $1\times10^6$ cells / cm2, the bioreactor (1) over a period of 30 to 180, preferably 60 to 120 min in one horizontal position and stored at rest, then the gas supply via the supply (5) and discharge (6) with a, a laminar and no swirling flow set up the amount of gas switched on, the cell-free culture medium is pumped at rates of 1 to 20 x / h, preferably 5 to 15 x / h, and samples are taken regularly via the sterile access (10), the glucose content falling to 1/3 of the Initial value in the culture medium is fed via the feed (8) fresh medium with a continuously increasing amount, so that the glucose content is> 1/3 of the initial value and the lactate content is <3 g /l, the amount of media in the bioreactor is not more than a factor of 3 compared to the amount of the cell suspension at the start of the cultivation increases and the ratio of the circulating amount of media to the amount of fresh medium supplied does not exceed 5 to 1.

**Revendications**

1. Bioréacteur à méandre pour l'expansion dynamique, la différenciation et la récolte de cellules en croissance non adhérentes, en particulier de mélangea ou de fractions isolées de cellules hématopoïétiques, de cellules souches et de cellules progénitrices d'origine humaine, **caractérisé en ce que** le bioréacteur à méandre se compose de préférence d'un récipient de bioréacteur rectangulaire (1) il y a un matériau polymère transparent, qui est stérile fermé avec un couvercle (2) et sur lequel les entrées et sorties (3, 4, 5, 6) sont disposées à différentes hauteurs pour l'écoulement du milieu et des gaz, la surface inférieure (12) de la cuve de bioréacteur (1) est de 20 à 500 cm$^2$, de préférence de 50 à 100 cm$^2$, et la surface Inférieure (12) est pourvue de parois de séparation en forme de bande (11) qui divisent la surface inférieure (12) et serpentent à travers la cuve de bioréacteur (1) pour forcer les gaz et le milieu est, la distance (A) des cloisons (11) les unes des autres et des côtés les parois d'extrémité d du récipient de bioréacteur (1) sont de 2 à 10 mm, de préférence de 4 à 5 mm et il y a un flux laminaire moyen en amont avec un nombre Froude <0,005, de préférence 0,002 et un flux inférieur dans les filets d'écoulement, dans les canaux formés par les parois de séparation (8) avec un nombre de joie de 0 ou proche de 0.

2. Bioréacteur à méandre selon la revendication 1-2, **caractérisé en ce que** le trop-plein (13) disposé au niveau de l'ouverture de connexion (6) est disposé réglable en hauteur pour le bouillon de cellule épuisé.

3. Bioréacteur à méandre selon les revendications 1 à 3, **caractérisé en ce que** via le trop-plein (13) pour le bouillon de cellules (avec dispositifs d'arrêt, non représentés), plusieurs, notamment 2 à 5, réacteurs à biofilm sont connectés en série de manière modulaire, le module aval étant un par le taux d'expansion de 1:10 à 1:50 plus grande surface de plancher (12) du boîtier de bioréacteur (1) par rapport à la surface de plancher (12) du boîtier de bioréacteur (1) du module en amont.

4. Procédé de différenciation dynamique et de récolte de cellules hématopoïétiques, en particulier de cellules tueuses naturelles (cellules NK), de lymphocytes T et de lymphocytes B selon la revendication 1, à l'exception des cellules souches embryonnaires humaines, **caractérisé en ce que** l'aliment (7) et la décharge (9) pour le circuit de milieu couvrant la quantité de cellules en suspension dans le milieu via l'alimentation (7) vers le récipient de bioréacteur positionné horizontalement (1) en un, un revêtement complet de la surface inférieure (12) du récipient de bioréacteur (1) et en un Couche cellulaire avec un contact étroit des cellules entre elles, imposant un nombre de cellules de 0,1 à 2 x106, de préférence de 0,5 à 1x106 cellules / cm2, le bioréacteur (1) sur une période de 30 à 180, de préférence de 60 à 120 min en une position horizontale et stockée au repos, puis l'alimentation en gaz via l'alimentation (5) et l'évacuation (6) avec a, un flux laminaire et sans turbulence la quantité de gaz allumé, le milieu de culture acellulalre est pompé à des vitesses de 1 à 20 x / h, de préférence de 5 à 15 x / h, et des échantillons sont prélevés régulièrement via l'accès stérile (10), la teneur en glucose tombant à 1/3 de la La valeur initiale dans le milieu de culture est alimentée via le milieu frais d'alimentation (8) avec une quantité en augmentation continue, de sorte que la teneur en glucose est> 1/3 de la valeur initiale et la teneur en lactate est <3 g/l, la quantité de milieu dans le bioréacteur n'est pas plus d'un facteur 3 par rapport à la quantité de suspension cellulaire au début de la culture augmente et le rapport entre la quantité de milieu en circulation et la quantité de milieu frais fourni ne dépasse pas 5 pour 1.

**Fig. 1**

unterschiedlich hohe
Anschlußöffnung für Medium/Gas

verbrauchtes
Medium

Frischmedium

Probenehmer

**Fig. 2**

t/mm

5 mm

freie Oberfläche

Sohle

w/mm/s

0,5 mm/s

Geschwindigkeitsprofil des Stromfadens in
Stromröhre

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009139413 A
- US 2007224174 A
- KR 100650384
- JP 4299983 B
- EP 0289896 A
- JP 63202378 A
- EP 0280054 A1
- DE 3875752 T2
- JP 63028388 A
- EP 0247613 A2
- KR 20070030155 A
- KR 20070030115 A
- DE 10223536 A1
- WO 2009151182 A1
- DE 3782736 T2
- DE 3788026
- FR 1448148
- WO 8700548 A
- DE 102007036611 A1
- US 5416022 A
- US 4220725 A
- US 4647539 A
- DE 3895414
- US 5290700 A
- DE 3409501
- DE 4229334
- WO 9318133 A
- DE 602004008453 T2
- EP 1625212 B1
- EP 1479760 A1
- FR 2608169

**In der Beschreibung aufgeführte Nicht-Patentliteratur**

- **LU.** *Critical Reve. Onco / Hematol,* 1996, vol. 22, 61-78
- *Journal Immunol Methods,* 30. Juni 2009, vol. 345 (1-2), 90-99